# EUROPÄISCHE PATENTSCHRIFT

(11) **EP 1 457 215 B1**
(45) Veröffentlichungstag und Bekanntmachung des Hinweises auf die Patenterteilung: **25.07.2007**
(21) Anmeldenummer: 04003446.4
(22) Anmeldetag: 17.02.2004
(51) Int. Cl.: A61L 29/04, A61M 25/00

(54) **Zweischichtiger Katheter mit verbesserten Gleiteigenschaften für die Einführungshilfe**
Double layer catheters with improved gliding properties for the guiding wire
Cathétère bi-couche avec des propriétés de glissement améliorées pour les fils guides

(30) Priorität: 13.03.2003 DE 20303982 U
(43) Veröffentlichungstag der Anmeldung: 15.09.2004
(73) Patentinhaber: RAUMEDIC AG, 95233 Helmbrechts (DE)
(72) Erfinder: Dolla, Andreas, 95032 Hof (DE)
(74) Vertreter: Hofmann, Matthias

(56) Entgegenhaltungen:
- EP-A- 0 650 740
- WO-A-94/01160
- WO-A-03/094989

## Beschreibung

Die Erfindung betrifft ein Set aus einer Einführungshilfe und einem Katheter, der mittels der Einführungshilfe positioniert wird, nach dem Oberbegriff des Anspruchs 1.

In der Urologie und der enteralen Ernährung wird eine Vielzahl von Kathetern eingesetzt, bei denen aus funktionalen Gründen weiche Rohstoffe, insbesondere Polyurethan, verwendet werden.

Die Applikation der Kathetersysteme, beispielsweise im Ureter (hier besonders die Ureter-Katheter und die Ureter-Dauerschienen) und im Magen/Darmtrakt (beispielsweise für die nasale Sondenernährung durch Einführung von Nährstoffen) erfolgt unter Anwendung von Einführungshilfen, beispielsweise einem Stahl-Mandrin als Führungsdraht aus korrosionsbeständigem Chrom-Nickel-Stahl oder einem PTFE-beschichteten Spiralmandrin. Der mit einem solchen Mandrin versteifte Katheter wird an die gewünschte Position vorgeschoben, anschließend wird die Einführungshilfe entfernt und verworfen. Hersteller solcher Kathetersysteme sind beispielsweise die Firmen Fresenius, Pfrimmer, Rüsch, B. Braun oder Nutrica.

Nachteilig an diesem Stand der Technik ist, dass sich die Einführungshilfe nach erfolgter Applikation des Katheters in vielen Fällen nicht mehr problemlos aus diesem entfernen lässt. Dies wirkt sich vor allem bei langen und dünnwandigen Kathetern aus. Ursächlich verantwortlich dafür ist, dass die weichen Rezepturen des Polyurethans im Shore-Härte-Bereich zwischen A 80 und 87 klebrig sind, so dass sich die Einführungshilfe teilweise nur unter erheblichem Kraftaufwand und bei gleichzeitiger Deformation des Katheters wieder entfernen lässt. Gelegentlich kann die Einführungshilfe überhaupt nicht mehr entfernt werden, so dass das gesamte Kathetersystem entfernt werden muss und eine neue Applikation erforderlich wird - eine unnötige Belastung des Patienten.

Zwar lassen sich die Auszugskräfte durch Beschichtung der Katheterinnenfläche, beispielsweise mit Hydrogelen, durch Beschichtung der Einführungshilfe, beispielsweise mit polymeren Fluorverbindungen, wie PTFE, oder durch Applikation von als Gleitmittel wirkenden Silikonölen auf den Einführungshilfen verringern. Eine sichere und problemlose Entfernung der Einführungshilfe ist jedoch trotzdem nicht immer gegeben.

Aus der EP 0 650 740 A1 (D3) ist ein Set aus einem Katheter mit einem Führungsdraht bekannt. Dort wird das Einführen des Führungsdrahtes erleichtert, indem ein Katheter mit einer Innenschicht aus einem Material mit geringem Reibungskoeffizienten bereitgestellt wird.

Die WO 94/01160 A1 zeigt einen Regionalanästhesie-Katheter mit einer weicheren Außenschicht und einer härteren Innenschicht.

Aufgabe der Erfindung war es, ein Set aus einem Mandrin und einem Katheter nach dem Oberbegriff des Anspruchs 1 bereitzustellen, das kostengünstig herstellbar ist, weitgehend auf bereits bestehenden Komponenten basiert - ohne dass auf kostspielige, oben beschriebene Maßnahmen der Gleitfähigkeitsverbesserung zurückgegriffen werden muss - und trotzdem eine sichere und problemlose Entfernung der Einführungshilfe aus dem Katheter ermöglicht.

Die Aufgabe konnte gelöst werden durch die Merkmale des Patentanspruches 1. Bevorzugte Ausführungsformen und Weiterbildungen finden sich in den Unteransprüchen.

Die Lösung der Aufgabe gelang durch ein Set mit dem im Kennzeichnungsteil des Anspruchs 1 angegebenen Merkmalen.

Bei dieser Lösung kann als Außenschicht das bisher eingesetzte Polyurethan der Shore-Härte A 80 bis A 87 verwendet werden, die Innenschicht des coextrudierten Katheters ist aus einem harten Polyurethan oder Polyamid ausgebildet. Hier kommen aromatische oder aliphatische Polyetherpolyurethane in Frage, ebenso wie Polyamide auf Basis Polyamid 11, Polyamid 12 oder Polyamid 6.

Wesentlich und erfindungsgemäß ist zusätzlich, dass die Shorehärte der Innenschicht im Bereich zwischen D 53 und D 75 liegt und dass deren Wandstärke entsprechend den Abmessungen des Katheters so abgestimmt ist, dass eine signifikante Änderung der Kathetersteifigkeit vermieden wird. Die Wandstärke der Innenschicht liegt daher vorzugsweise unter 0,1 mm.

Die nachfolgende Tabelle 1 zeigt beispielhaft die Wanddicke von erfindungsgemäßen Kathetern mit einer Außenschicht aus PUR der Shore-Härte A 80 und einer coextrudierten Innenschicht aus PUR der Shore-Härte D 68 im Bezug zur jeweiligen Kathetergröße und die sich daraus ergebenden Katheter-Außenabmessung.

**TABELLE 1**

| Katheter-Größe | Außenabmessung des Katheters (mm) | Wanddicke der Innenschicht (mm) |
|---|---|---|
| CH 4 | 1,33 | 0,03 |
| CH 5 | 1,63 | 0,04 |
| CH 6 | 2,00 | 0,04 |
| CH 7 | 2,33 | 0,05 |
| CH 8 | 2,67 | 0,06 |
| CH 9 | 3,00 | 0,07 |
| CH 10 | 3,33 | 0,07 |
| CH 12 | 4,00 | 0,09 |
| CH 14 | 4,67 | 0,10 |

Der erfindungsgemäße Katheter erfüllt die anwendungstechnischen Voraussetzungen für die Applikation Urologie (beispielsweise für Ureterkatheter oder Ureterdauerschienen) oder enterale Ernährung (beispielsweise für nasale Sonden) hinsichtlich seiner chemischen Beständigkeit gegenüber Nährlösungen oder Harn.

Besonders vorteilhaft an den erfindungsgemäßen Kathetern sind vor allem, wie im nachfolgenden Ausführungsbeispiel gezeigt wird, die wesentlich geringeren Auszugskräfte für die Entfernung der Einführungshilfe aus dem Katheter.
Überraschender Weise bringt diese harte Innenschicht des Katheters aus PUR oder PA der Shore-Härte D 53 - 75 im System Katheter - Einführungshilfe ein wesentlich besseres Gleitverhalten, als die üblichen Systeme, wie zum Beispiel:
- Katheter aus PUR der Shore-Härte A 80 bis A 87 mit einer Hydrogel-Innenbeschichtung und Einführungshilfe aus Stahl, mit oder ohne PTFE-Beschichtung
   oder
- Katheter aus PUR der Shore-Härte A 80 bis A 87 mit einer mit Siliconöl versehenen Einführungshilfe aus Stahl mit oder ohne PTFE-Beschichtung.

Vorteilhaft an der Erfindung ist auch, dass eine röntgenkontrastfähige Ausführung mit z.B. 20-40% BaSO4 als Füllstoff nun möglich ist, die über eine glatte Innenoberfläche verfügt.
Herkömmliche, mit Bariumsulfat gefüllte sogenannte Vollkontrastschläuche verfügen nämlich über ein raue Innenoberfläche, die zur Inkrustation bei den urologischen Kathetern und zur Verstopfung bei den Ernährungssonden führen kann.
Durch die erfindungsgemäße Ausführung als coextrudierter Katheter mit einer gefüllten Außenschicht und einer ungefüllten Innenschicht können diese Verkrustungen bzw Verstopfungen deutlich reduziert werden.

Im nachfolgenden Ausführungsbeispiel werden die wesentlichen Kathetereigenschaften für die Urologie oder die enterale Ernährung, nämlich Auszugskraft, Einführungshilfe und Kathetersteifigkeit, eines herkömmlichen PUR-Katheters dem erfindungsgemäßen Katheter gegenübergestellt.

### Ausführungsbeispiel

In einer Extrusionsstrecke mit einem Haupt- und einem Zuspritzextruder wird PUR zu einem coextrudierten Katheter geformt. Die Außenschicht hat eine Shore-Härte von A 80, die Innenschicht eine Shore-Härte von D 55.
Die Abmessungen des Katheters mit der Größe CH 5 für eine Ureterdauerschiene sind folgende:
- Dicke der Außenschicht: 0,31 mm
- Dicke der Innenschicht: 0,04 mm
- Außendurchmesser: 1,63 mm
- Länge: 300 mm

Eine PTFE-beschichtete Einführungshilfe mit einem Außendurchmesser von 0,69 mm führt man in den Katheter ein und zieht sie nach einer Wartezeit von 10 Minuten wieder heraus.
Die Auszugskraft wird mit einer Zwick-Universalprüfmaschine bei 23° C gemessen.

Zum Vergleich ist der Erfindung in der nachfolgenden Tabelle 2 als Vergleichsbeispiel ein konventioneller PUR-Einschichtkatheter gegenüber gestellt:

**TABELLE 2**

| | Erfindungsgemäßer Katheter | Vergleichsbeispiel |
|---|---|---|
| Wandstärke | Gesamt 0,35 mm | Gesamt 0,35 mm |
| Auszugskraft | Kleiner 1 N | 4-6 N (Spitzenwerte bis 8,5 N) *) |
| Kathetersteifigkeit | 97 mN | 91 mN |

| | | |
|---|---|---|
| *) Die starke Streuung der Messwerte rührt daher, dass - bedingt durch starkes Haften der Einführungshilfe an der Katheterwand - ein ruckartiges Entfernen des Führungsdrahtes aus dem Katheter auftritt (stick-slip-Effekt). | | |

Die Probleme beim Herausziehen der Einführungshilfe steigen mit Erhöhung der Temperatur.
Entsprechende Versuche zeigten, dass in 2 von 5 Fällen bei Kathetern nach dem Stand der Technik, bei denen - zur Simulation der Körpertemperatur - das Ausziehverhalten bei 37°C getestet wurde, die Einführungshilfe nicht mehr zu entfernen war.
Im Falle des erfindungsgemäßen Katheters war bei der Prüfung bei 37 °C ein stick-slip-Effekt nicht zu beobachten, allerdings stieg die Auszugskraft auf 3 N an.

## Patentansprüche

1. Set aus
- einer Einführungshilfe und
- einem Katheter,
-- der mittels der Einführungshilfe positioniert wird und
-- aus einer Innenschicht und einer Außenschicht besteht,
**dadurch gekennzeichnet, dass**
- der Katheder zum Einsatz in der Urologie oder der enteralen Emärung ausgebildet ist,
- die Außenschicht eine Shorehärte zwischen A 80 und A 87 aufweist,
- die Innenschicht eine Shorehärte zwischen D 53 und D 75 und eine Wandstärke von 0,1 mm und darunter aufweist.

2. Set nach Anspruch 1, **dadurch gekennzeichnet, dass** die Innenschicht ausgewählt ist aus der Gruppe Polyamid 11, Polyamid 12, Polyamid 6 oder Polyurethan.

3. Set nach Anspruch 1, **dadurch gekennzeichnet, dass** die Außenschicht aus Polyurethan besteht.

4. Set nach Anspruch 1, **dadurch gekennzeichnet, dass** die Außenschicht ganz oder teilweise im Röntgen sichtbar ist.

5. Set nach Anspruch 1, **dadurch gekennzeichnet, dass** die Außenschicht Füllstoffe enthält.

## Claims

1. Set comprising an insertion aid and a catheter, which is positioned by means of the insertion aid and consists of an inner layer and an outer layer, **characterised in that** the catheter is configured for use in urology or enteral feeding, the outer layer has a Shore hardness between A 80 and A 87 and the inner layer has a Shore hardness between D 53 and D75 and a wall thickness of 0.1 mm or less.

2. Set according to claim 1, **characterised in that** the inner layer is selected from the group consisting of polyamide 11, polyamide 12, polyamide 6 or polyurethane.

3. Set according to claim 1, **characterised in that** the outer layer consists of polyurethane.

4. Set according to claim 1, **characterised in that** the outer layer is either entirely or partially visible in X-ray.

5. Set according to claim 1, **characterised in that** the outer layer contains fillers.

## Revendications

1. Ensemble formé par
- un fil guide,
- un cathéter,
qui est mis en place au moyen d'un fil guide, et
qui est formé par une couche intérieure et une couche extérieure,
**caractérisé en ce que**
- le cathéter est réalisé pour être utilisé en urologie ou pour l'alimentation entérale,
- la couche extérieure présente une dureté Shore entre A 80 et A 87,
- la couche intérieure a une dureté Shore entre D 53 et D 75, et une épaisseur de paroi de 0,1 mm et moins.

2. Ensemble selon la revendication 1, **caractérisé en ce que** la couche intérieure est choisie dans le groupe polyamide 11, polyamide 12, polyamide 6 ou polyuréthanne.

3. Ensemble selon la revendication 1, **caractérisé en ce que** la couche extérieure est réalisée en polyuréthanne.

4. Ensemble selon la revendication 1, **caractérisé en ce que** la couche extérieure est visible totalement ou partiellement en radiologie.

5. Ensemble selon la revendication 1, **caractérisé en ce que** la couche extérieure contient des matières de charge.
